# FASCICULE DE BREVET EUROPEEN

(11) **EP 1 097 695 B1**
(45) Date de publication et mention de la délivrance du brevet: **18.09.2002**
(21) Numéro de dépôt: 00402798.3
(22) Date de dépôt: 10.10.2000
(51) Int. Cl.: A61K 7/00, A61K 7/02, A61K 7/06, A61K 7/48, C08J 3/03, C08L 83/04

(54) **Composition contenant une suspension aqueuse de particules d'un organopolysiloxane élastomère, et ses utilisations cosmétiques**
Wässrige Suspension von Organopolysiloxanelastomerpartikeln und ihre Verwendung
Composition containing an aqueous suspension of particles of an organopolysiloxane elastomer and cosmetical uses thereof

(30) Priorité: 08.11.1999 FR 9913992
(43) Date de publication de la demande: 09.05.2001
(73) Titulaire: L'OREAL, 75008 Paris (FR)
(72) Inventeur: Lorant, Raluca, 94320 Thiais (FR)
(74) Mandataire: Rasson, Catherine

(56) Documents cités:
- EP-A- 0 765 656
- FR-A- 2 757 380
- FR-A- 2 758 985
- FR-A- 2 768 926
- US-A- 5 928 660

## Description

La présente invention se rapporte à une composition comprenant une phase aqueuse et une phase huileuse, au moins un tensioactif de HLB inférieur ou égal à 7, et une suspension aqueuse de particules d'organopolysiloxane élastomère au moins partiellement réticulé. L'invention se rapporte aussi aux utilisations notamment cosmétiques de cette composition, en particulier pour le soin, le maquillage, le nettoyage et/ou le démaquillage de la peau, des muqueuses, des yeux et/ou des cheveux, ainsi que pour le traitement des peaux sèches.

Dans le domaine cosmétique, il est courant d'utiliser des crèmes constituées d'une émulsion eau-dans-huile (E/H) comportant une phase aqueuse dispersée dans une phase huileuse. Ces émulsions comportent une phase continue huileuse et permettent donc de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches.

Toutefois, les émulsions contenant une forte teneur en huile, et plus spécialement celles à phase huileuse continue, ont tendance à laisser sur la peau un film au toucher gras, collant ou poisseux, s'accompagnant souvent d'un aspect brillant, les rendant peu attrayantes, voire d'utilisation rédhibitoire, surtout lorsqu'il s'agit d'applications répétées sur le cuir chevelu ou le visage, et plus spécialement pour les utilisateurs ou les patients à peau mixte, grasse ou à peau acnéique. De plus, la présence d'une grande quantité de corps gras, rend difficile l'étalement de la composition sur la peau. Or, certains actifs cosmétiques et/ou dermatologiques ne peuvent être formulés qu'en présence d'une grande quantité de corps gras et notamment dans un milieu anhydre ou à phase huileuse continue. Dans une moindre mesure, ce problème d'effet gras peut se poser aussi pour des émulsions H/E.

En outre, certaines compositions destinées aux peaux sèches, voire très sèches, nécessitent la présence d'une phase huileuse continue et/ou d'une phase huileuse continue (émulsion E/H) ou dispersée (émulsion H/E) contenant des huiles minérales telles que l'huile de vaseline, les huiles minérales étant particulièrement efficaces pour la réparation de l'effet barrière et donc le traitement des peaux sèches. Toutefois, ces huiles minérales augmentent encore l'effet gras des émulsions les contenant.

Par ailleurs, les émulsions E/H sont fréquemment préparées avec, comme tensioactifs, des organopolysiloxanes oxyalkylénés (tensioactifs siliconés) tels que les organopolysiloxanes solides élastomères réticulés comportant au moins un groupement oxyalkyléné, introduits sous forme de mélange avec une phase grasse qui peut amener encore un effet gras à la composition.

Il subsiste donc le besoin d'une composition notamment cosmétique et/ou dermatologique, sous forme d'émulsion comportant une phase huileuse en une quantité suffisante pour être susceptible de contenir des actifs lipophiles et/ou contenant des huiles minérales et/ou un organopolysiloxane oxyalkyléné, mais ne conférant pas de sensation de gras et/ou de collant sur la peau ni d'aspect brillant.

De façon surprenante, la demanderesse a trouvé qu'il était possible de supprimer le toucher gras et/ou collant sur la peau d'une émulsion, en introduisant dans la phase aqueuse une suspension aqueuse de particules d'un organopolysiloxane élastomère.

L'invention a donc pour objet une émulsion non collante et non grasse, contenant un tensioactif éventuellement siliconé et une dispersion aqueuse de particules d'organopolysiloxane élastomère.

Plus précisément, l'invention se rapporte à une composition sous forme d'émulsion eau-dans-huile, comprenant une phase aqueuse dispersée dans une phase huileuse, caractérisée en ce qu'elle contient au moins un tensioactif de HLB inférieur ou égal à 7, en ce que la phase huileuse représente au moins 5 % en poids par rapport au poids total de la composition, et en ce que la phase aqueuse contient une suspension aqueuse de particules d'organopolysiloxane élastomère au moins partiellement réticulé.

L'invention se rapporte aussi à une composition comprenant une phase aqueuse et une phase huileuse, l'une des phases étant dispersée dans l'autre, caractérisée en ce qu'elle contient au moins un organopolysiloxane solide élastomère réticulé comportant au moins un groupement oxyalkyléné, et en ce que la phase aqueuse contient une suspension aqueuse de particules d'organopolysiloxane élastomère au moins partiellement réticulé.

La présence de la suspension aqueuse de particules d'organopolysiloxane élastomère partiellement réticulé permet d'éviter l'effet collant et/ou gras apporté par la grande quantité de corps gras et/ou la présence d'huiles minérales et/ou de corps gras cireux ou pâteux qui ont un rôle d'actifs réparateurs des peaux sèches comme c'est le cas de la vaseline ou du beurre de karité par exemple. Cette propriété est apportée notamment du fait que les particules d'élastomère sont en suspension aqueuse et sont présentes dans la phase aqueuse.

Aussi, la présente invention se rapporte encore à l'utilisation d'une suspension aqueuse de particules d'organopolysiloxane élastomère au moins partiellement réticulé, dans la phase aqueuse d'une composition sous forme d'émulsion eau-dans-huile contenant au moins 5 % en poids de phase huileuse par rapport au poids total de la composition, afin d'éliminer le toucher collant et/ou gras dû à cette phase huileuse.

Par « élastomère» on entend un matériau souple, déformable ayant des propriétés viscoélastiques et notamment la consistance d'une éponge ou d'une sphère souple. Son modèle d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes élastomères de la composition de l'invention ne sont pas desséchants pour la peau et apportent de bonnes propriétés cosmétiques, notamment de douceur, de fraîcheur et de matité. Ces élastomères conduisent à des compositions confortables à l'application, de bon étalement, douces, non collantes au toucher et non grasses.

Les organopolysiloxanes élastomères conformes à l'invention sont partiellement ou totalement réticulés et de structure tridimensionnelle. Ils peuvent être choisis notamment parmi les polymères réticulés décrits dans la demande JP-A-10/175816 (correspondant à US-A-5,928,660). Ce document décrit la préparation de ces polymères et leur utilisation dans des compositions cosmétiques, mais il ne décrit ni ne suggère que ces polymères puissent être utilisés dans la phase aqueuse d'une émulsion eau-dans-huile pour éviter l'effet collant apporté par la phase huileuse de l'émulsion.

EP-A-0 765 656 divulgue des émulsions cosmétiques eau-dans-huile comprenant un mélange de poudres dont un organopolysiloxane élastomère et de la silice hydrophobe ainsi qu'un émulsifiant à HLB inférieur ou égal à 7.

FR-A-2 768 926 décrit des compositions cosmétiques contenant une phase grasse liquide associée à une phase solide comprenant des particules d'organopolysiloxane élastomérique réticulé pouvant se présenter sous forme d'un gel anhydre, d'une émulsion eau-dans-huile ou huile-dans-eau.

Selon cette demande, ils sont obtenus par réaction d'addition et de réticulation, en présence d'un catalyseur du type platine, d'au moins :
- (a) un organopolysiloxane (i) ayant au moins deux groupes vinyliques en position α-ω de la chaîne siliconée ; et
- (b) un organopolysiloxane (ii) ayant au moins un atome d'hydrogène lié à un atome de silicium par molécule.

En particulier, l'organopolysiloxane (I) est choisi parmi les polydiméthylsiloxanes et est plus spécifiquement un α-ω-diméthylvinyl polydiméthylsiloxane.

La suspension aqueuse de particules d'organopolysiloxane élastomère, utilisée dans la composition selon l'invention peut être notamment obtenue comme suit :
- (a) mélange de l'organopolysiloxane (i) et de l'organosiloxane (ii) ;
- (b) ajout de la phase aqueuse contenant un émulsifiant au mélange de l'étape (a);
- (c) émulsification de la phase aqueuse et dudit mélange ;
- (d) ajout d'eau chaude à l'émulsion de la phase (c) ; et
- (e) polymérisation de l'organopolysiloxane (i) et de l'organosiloxane (ii) en émulsion en présence d'un catalyseur de platine.

Dans le procédé décrit ci-dessus, l'eau est avantageusement ajoutée à une température d'environ 40 à 60°C. Après l'étape (e), il est possible de sécher les particules obtenues, pour en évaporer toute ou partie de l'eau piégée.

En particulier, les particules d'organopolysiloxane élastomère (en matière active) ont une taille allant de 0,1 à 500 µm et mieux de 3 à 200 µm. Ces particules peuvent être sphériques, plates ou amorphes avec, de préférence, une forme sphérique.

Ainsi qu'indiqué dans le procédé décrit ci-dessus, ces particules d'organopolysiloxane, pour se disperser de façon stable dans l'eau, peuvent être associées à un ou plusieurs émulsifiants non ioniques, cationiques ou anioniques de HLB (Hydrophilic lipophilic balance) supérieur ou égal à 8. La proportion d'émulsifiants dans la suspension aqueuse va de préférence de 0,1 à 20 parties en poids pour 100 parties en poids de la suspension aqueuse d'organopolysiloxane élastomère, et mieux, de 0,5 à 10 parties en poids (voir description du document JP-A-10/175816).

En outre, à ces particules d'organopolysiloxane élastomère, peuvent être associées dans la suspension aqueuse, des corps gras, et notamment des huiles telles que celles décrites dans le document JP-A-10/175816, des cires ou des gommes solides à température ambiante, des corps gras pâteux, d'origine animale, végétale, minérale ou synthétique, leurs mélanges ainsi que des poudres inorganiques telles que celles décrites dans ce document.

Les suspensions aqueuses de particules d'organopolysiloxanes de la composition de l'invention sont par exemple celles commercialisées sous les noms BY 29-122 et BY 29-119 par la société Dow Corning Electric. On peut aussi utiliser un mélange de ces produits commerciaux. Ces suspensions comportent environ 63 % en poids de particules d'organopolysiloxane élastomère (donc environ 63 % de matière active) par rapport au poids total de la suspension.

De façon préférentielle, les particules d'organopolysiloxane élastomère sont présentes dans la composition de l'invention en une quantité en matière active allant par exemple de 0,1 à 30 % en poids et de préférence de 0,5 à 20 % en poids par rapport au poids total de la composition.

La composition de l'invention contient au moins 5 % en poids, et de préférence de 10 à 80 %, et mieux de 20 à 60 % en poids de phase huileuse, par rapport au poids total de la composition. On entend par « phase huileuse » dans la présente demande une phase grasse qui contient au moins une huile, c'est-à-dire un corps gras liquide à la température ambiante (20-30°C) et tout autre composé lipophile. La nature de la phase huileuse de la composition selon l'invention n'est pas critique. La phase huileuse peut ainsi être constituée par tous les corps gras et notamment les huiles, classiquement utilisés dans les domaines cosmétique ou dermatologique.

Parmi les huiles utilisables dans la composition de l'invention, on peut citer par exemple les huiles d'origine végétale, telles que les huiles de jojoba, avocat, amande douce, abricot, maïs, le beurre de karité et la fraction liquide de beurre de karité, le beurre de Shorea ; les huiles minérales comme l'huile de vaseline ; les huiles de synthèse comme les triglycérides (par exemple caprylic/capric triglycerides), le palmitate d'éthyl-2 hexyle, le myristate d'isopropyle, l'isoparaffine hydrogénée, l'isononanoate d'isononyle, l'octanoate de cétéaryle ; les huiles de silicone volatiles (cyclométhicones) ou non volatiles et les huiles fluorées. Les autres corps gras susceptibles d'être présents dans la phase huileuse peuvent être par exemple les acides gras tels que l'acide stéarique, les alcools gras tels que l'alcool stéarylique, et les cires.

Ainsi qu'indiqué plus haut, il est particulièrement approprié d'utiliser au moins une huile minérale pour obtenir une composition adaptée aux peaux sèches. Aussi, selon un mode particulier de réalisation de l'invention, la composition contient au moins une huile minérale, de préférence en une quantité allant de 1 à 100 % du poids total de la phase huileuse.

Le tensioactif de HLB (Hydrophilic Lipophilic Balance ou Balance hydrophile lipophile) inférieur ou égal à 7 peut être tout tensioactif approprié pour les émulsions E/H, et en particulier les tensioactifs siliconés (organopolysiloxanes oxyalkylénés), non ioniques ou anioniques. Il peut être choisi notamment parmi les organopolysiloxanes oxyalkylénés, les alkylpolyglycosides, les esters d'acide gras et de polyol et leurs mélanges dans la mesure où ces tensioactifs ou leurs mélanges ont un HLB inférieur ou égal à 7.

Comme organopolysiloxanes oxyalkylénés pouvant entrer dans la composition selon l'invention, on peut utiliser notamment les dimethicone copolyols, les alkyldiméthicone copolyols et les organopolysiloxanes solides élastomères réticulés comportant au moins un groupement oxyalkyléné.

Comme dimethicone copolyol, on peut citer par exemple le mélange de dimethicone copolyol, de cyclomethicone et d'eau (10/88/2), commercialisé par la société Dow Corning sous la dénomination DC3225C ou DC2-5225C, et le mélange de dimethicone copolyol et de cyclopentasiloxane (85/15) commercialisé sous la dénomination Abil EM-97 par la société Goldschmidt.

Comme alkyldiméthicone copolyol, on peut citer notamment ceux ayant un radical alkyle comportant de 10 à 22 atomes de carbone, tel que le cétyl diméthicone copolyol comme le produit commercialisé sous la dénomination Abil EM-90 par la société Goldschmidt ; le lauryl diméthicone copolyol et par exemple le mélange d'environ 91 % de lauryl diméthicone copolyol et d'environ 9 % d'alcool isostéarylique, commercialisé sous la dénomination Q2-5200 par la société Dow Corning, et les mélanges de ces polydiméthylsiloxanes oxyalkylénés.

Les organopolysiloxanes solides élastomères réticulés comportant au moins un groupement oxyalkyléné, pouvant être utilisés comme tensioactifs dans la composition de l'invention, peuvent contenir un ou plusieurs groupements oxyalkylénés et en particulier oxyéthylénés (OE), par exemple de 1 à 40 motifs oxyalkylénés et mieux 1 à 20 motifs oxyalkylénés, pouvant former des chaînes polyoxyalkylènes et notamment polyoxyéthylènes. Ces groupements peuvent être pendants, en bout de chaîne ou destinés à lier deux parties de la structure siliconée. Les atomes de silicium portant ces groupements sont au nombre d'environ 1 à 10.

Bien que ces organopolysiloxanes aient de préférence des groupement(s) oxyéthyléné(s), ils peuvent aussi être à groupement(s) oxypropyléné(s). Ces organopolysiloxanes peuvent aussi comporter à la fois un ou plusieurs groupement(s) oxyéthyléné(s), 1 à 20 (OE) par exemple, et un ou plusieurs groupement(s) oxypropyléné(s) (OP), 0 à 20 par exemple ; ces organopolysiloxanes sont appelés aussi des organopolysiloxanes à groupement(s) alkyléthoxy-propyléné(s). De préférence, le nombre de groupements oxyéthylénés est supérieur au nombre de groupements oxypropylénés.

Ces organopolysiloxanes élastomères sont partiellement ou totalement réticulés et de structure tridimensionnelle. Ils peuvent se présenter sous forme de poudre, les particules constituant cette poudre ayant une taille allant généralement de 0,1 à 500 µm et mieux de 3 à 200 µm et pouvant être sphériques, plates ou amorphes avec, de préférence, une forme sphérique. Ils peuvent aussi se présenter sous forme de gel contenant l'organopolysiloxane élastomère dispersé dans une phase huileuse. Cette phase huileuse, appelée encore phase grasse liquide, peut comprendre tout corps non aqueux ou mélange de corps non aqueux, liquide à température ambiante (environ 25°C).

Les organopolysiloxanes pouvant être utilisés dans la composition de l'invention comme tensioactifs sont en particulier obtenus selon le mode opératoire des exemples 3, 4 et 8 du document US-A-5,412,004 et des exemples du document US-A-5,811,487.

Il s'agit par exemple de celui commercialisé sous la référence KSG 21 par la société Shin Etsu ou du produit de l'exemple 3 (exemple de synthèse) du brevet US-A-5,412,004.

Le KSG 21 se présente sous forme d'un gel et comprend 28 % d'organopolysiloxane et 72 % d'huile de silicone (PDMS) ayant une viscosité de 6 cSt.

Le produit de l'exemple 3 (exemple de synthèse) du document US-A-5,412,004 se présente sous la forme d'un gel pâteux contenant environ 32-33 % en poids d'organopolysiloxane réticulé à groupement(s) oxyéthyléné(s) et environ 67-68 % de PDMS 6 cSt. L'organopolysiloxane contient environ 18 % d'oxyde d'éthylène en poids par rapport au poids total du polymère.

Les alkylpolyglycosides pouvant être utilisés selon l'invention et ayant un HLB égal ou inférieur à 7 sont représentés par la formule générale (Il) suivante :

R-O-(G)ₓ (I)

dans laquelle R représente un radical alkyle ramifié et/ou insaturé, comportant de 14 à 22 atomes de carbone, G représente un sucre réduit comportant de 5 à 6 atomes de carbone, et x désigne une valeur allant de 1 à 10 et de préférence de 1 à 4.

G désigne notamment le glucose, le fructose ou le galactose.

Le radical alkyle insaturé peut comprendre une ou plusieurs insaturations éthyléniques, et en particulier une ou deux insaturations éthylèniques. Il comporte de préférence de 16 à 20 atomes de carbone, et mieux 18 atomes de carbone.

Les alkylpolyglycosides préférés selon la présente invention sont des composés de formule (II) dans laquelle R représente plus particulièrement un radical oléyle (radical insaturé en C18) ou isostéaryle (radical saturé en C18), G désigne le glucose, x est une valeur allant de 1 à 2.

L'alkylpolyglycoside utilisé dans l'émulsion de l'invention est de préférence choisi dans le groupe comprenant l'isostéaryl-glucoside, l'oléyl-glucoside et leurs mélanges.

Selon un mode particulier de réalisation de l'invention, l'alkylpolyglucoside indiqué ci-dessus peut être utilisé en mélange avec un co-émulsionnant. De manière avantageuse, le co-émulsionnant peut être choisi parmi les alcools gras et notamment ceux ayant la même chaîne grasse que celle de l'alkylpolyglucoside, c'est-à-dire comportant de 14 à 22 atomes de carbone et ayant une chaîne ramifiée et/ou insaturée, et par exemple l'alcool isostéarylique quand l'alkylpolyglucoside est l'isostéaryl-glucoside, et l'alcool oléylique quand l'alkylpolyglucoside est l'oleyl-glucoside.

Selon un mode préféré de réalisation de l'invention, l'alkylpolyglucoside utilisé dans l'émulsion de la présente invention est obtenu en mélange avec l'alcool gras correspondant sous forme d'une composition autoémulsionnante, comme décrit par exemple dans le document WO-A-92/06778.

Comme esters d'acide gras et de polyol pouvant entrer dans la composition selon l'invention, on entend ici aussi bien les esters proprement dits que leurs dérivés oxyalkylénés et notamment oxyéthylénés. Comme esters d'acide gras et de polyol, on peut citer par exemple les esters de sorbitan et d'acide gras, ce dernier étant par exemple l'acide stéarique, l'acide isostéarique, l'acide oléique, comme l'oléate de sorbitan (Span 80 de la société ICI), le sesqui-oléate de sorbitan (Arlacel 83 de la société ICI), le trioléate de sorbitan (Arlacel 85 de la société ICI) ; les esters de sucre, et notamment de glucose, et d'acides gras comme le sesqui-stéarate de méthylglucose (glucate SS de la société Amerchol), le dioléate de méthylglucose (Glucate DO de la société Amerchol) ; les esters gras polyglycérolés comme le deca-oléate de polyglyceryl 10 (Atlas SCS 2054 de la société ICI), l'isostéarate de polyglyceryl-4 (Isolan GI 34 de la société Goldschmidt), le succinate d'isostéaryl diglyceryl (Imwitor 780 K de la société Huls) ; les mélanges tels que l'isostéarate de glyceryl et de sorbitan oxyéthyléné commercialisé sous la dénomination Arlacel 582 par la société ICI, le mélange d'isostéarate de sorbitan et d'isostéarate de polyglycéryl-3, commercialisé sous la dénomination Arlacel 1690 par la société ICI, le mélange d'oléate de polyglycéryl-4 et de cocoate de propylène glycol oxyethyléné (8 OE) commercialisé sous la dénomination Emulsynt 1055 par la société ISP.

Comme tensioactif anionique, on peut citer par exemple le mélange de lanolate de magnésium avec l'huile de vaseline (Mexanyl GO de la société Chimex), le mélange de stéarate d'aluminium avec l'acide stéarique et le sulfate de sodium (Noremulsol G 5 de la société Verley).

Selon un mode préféré de réalisation de l'invention, le ou les tensioactifs sont choisis parmi les dimethicone copolyols, les alkyldiméthicone copolyols, les organopolysiloxanes solides élastomères réticulés comportant au moins un groupement oxyalkyléné, l'isostéaryl-glucoside, l'oléyl-glucoside et leurs mélanges.

La composition de l'invention comprend généralement une quantité de tensioactif allant de 0,5 à 20 % en poids de matière active, et de préférence de 1 à 10 % en poids de matière active par rapport au poids total de la composition.

La composition selon l'invention peut être appropriée notamment pour une utilisation topique et constituer en particulier une composition cosmétique et/ou dermatologique. Elle contient alors un milieu physiologiquement acceptable. On entend par "physiologiquement acceptable" un milieu compatible avec la peau, les yeux et les fibres kératiniques des êtres humains.

La composition de l'invention peut être plus ou moins fluide, et elle peut avoir par exemple l'aspect d'un lait ou d'une crème.

De façon connue, les compositions de l'invention peuvent contenir des adjuvants habituels dans les domaines considérés, tels que des actifs hydrophiles ou lipophiles, des gélifiants hydrophiles ou lipophiles, des conservateurs, des antioxydants, des parfums, des solvants, des charges, des filtres, des agents basiques ou acides et encore des vésicules lipidiques. Ces adjuvants sont utilisés dans les proportions habituelles dans les domaines considérés, et par exemple de 0,01 à 20 % du poids total de la composition, et ils sont, selon leur nature, introduits dans l'une ou l'autre phase de la composition, ou encore dans des vésicules. Ces adjuvants ainsi que leurs concentrations doivent être tels qu'ils ne modifient pas la propriété recherchée pour l'émulsion de l'invention.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles. On peut utiliser notamment le produit vendu sous le nom de SEPIGEL 305 par la société Seppic (nom CTFA : polyacrylamide/C13-14 isoparaffin/Laureth-7) et le produit vendu sous le nom de HOSTACERIN AMPS par la société Hoechst (nom CTFA : Ammonium polyacryldimethyltauramide).

Comme gélifiants lipophiles, on peut citer les argiles modifiées comme les bentones, les sels métalliques d'acides gras, la silice hydrophobe et les polyéthylènes.

Comme actifs, on peut citer notamment les filtres solaires ; les vitamines et notamment les vitamines A (rétinol), C (acide ascorbique), E (tocophérol), B3 (niacinamide), F et D et leurs dérivés ; les acides gras insaturés comme l'acide linoléique et l'acide linolénique ; l'alpha-bisabolol ; les beurres d'origine végétale citées ci-dessus parmi les huiles, comme le beurre de Shorea ou le beurre de karité qui reconstituent la barrière lipidique de la peau et permettent le traitement des peaux sèches ; l'urée ; la rutine ; les enzymes ; les extraits naturels tels que le thé vert, l'extrait de mélisse, l'extrait de thym, les oligomères procyannidoliques (OPC) tels que l'OPC d'aubépine, l'OPC de pin et l'OPC de raisin ; certains acides tels que l'acide kojique, l'acide caféique, l'acide rétinoique et ses dérivés, l'acide benzène 1,4-di-(3-méthylidène 10-camphosulfonique) ; les caroténoïdes tels que les carotènes comme par exemple les α-, β- et γ-carotènes, le β,ϕ-carotène, le ξ-carotène, le β,λ-carotène, le lycopène (ψ,ψ-carotène), et leurs mélanges.

Les compositions, objets de l'invention, trouvent notamment leur application dans un grand nombre de traitements cosmétiques de la peau, des lèvres et des cheveux, y compris le cuir chevelu, notamment pour le traitement, la protection, le soin, le démaquillage et/ou le nettoyage de la peau, des lèvres et/ou des cheveux. Elles peuvent être destinées aussi au traitement des peaux sèches et/ou des lèvres sèches et/ou les peaux sensibles.

Les compositions selon l'invention peuvent par exemple être utilisées comme produits de soin, de démaquillage et/ou de nettoyage pour le visage sous forme de crèmes ou de laits.

Aussi, l'invention a encore pour objet l'utilisation cosmétique de la composition telle que définie ci-dessus pour le traitement, la protection, le soin, le démaquillage et/ou le nettoyage de la peau, des lèvres et/ou des cheveux.

L'invention a aussi pour objet un procédé de traitement cosmétique de la peau, y compris du cuir chevelu, des cheveux, et/ou des lèvres, caractérisé par le fait qu'on applique sur la peau, les cheveux et/ou les lèvres, une composition telle que définie ci-dessus.

L'invention a aussi pour objet l'utilisation de la composition telle que définie ci-dessus pour la fabrication d'une composition destinée au soin des peaux sèches et/ou des lèvres sèches et/ou des peaux sensibles.

Les exemples de compositions données ci-après sont donnés à titre illustratif. Les quantités sont données en pourcentage en poids, sauf mention contraire.

### Exemple 1 : Crème de soin nourrissante pour peaux sèches et sensibles

| *Phase huileuse :* | |
|---|---|
| Isostéaryl-glucoside/alcool isostéarylique (15/85) (soit 0,45 % de matière active d'alkyl-polyglucoside) | 3 % |
| Huile de noyaux d'abricot | 8 % |
| Cyclohexaméthicone | 8 % |
| Cires | 2 % |
| Vitamine E | 1 % |

| *Phase aqueuse :* | |
|---|---|
| Glycérine | 5 % |
| Sulfate de magnésium | 0,5 % |
| Conservateurs | 0,4 % |
| BY 29 (à 63 % de matière active) | 4 % |
| Eau | qsp 100 % |

Mode opératoire : on chauffe séparément à 70°C les phases huileuse et aqueuse, puis on émulsionne en dispersant sous agitation vigoureuse la phase aqueuse dans la phase huileuse.

On obtient ainsi une crème onctueuse, douce et riche à l'application. Elle traite les peaux sèches en apportant immédiatement un effet nutritif apaisant.

Malgré le fort taux de corps gras qui forment un film réparateur, cette crème n'est ni collante ni grasse.

### Exemple 2 : Crème de soin apaisante réparatrice pour peaux atopiques

| *Phase huileuse :* | |
|---|---|
| Cétyl dimethicone copolyol | 3 % |
| Huile de soja | 8 % |
| Cyclohexaméthicone | 8 % |
| Beurre de karité | 4 % |
| Vitamine E | 1 % |

| *Phase aqueuse :* | |
|---|---|
| Glycérine | 5 % |
| Sulfate de magnésium | 0,5 % |
| Conservateurs | 0,4 % |
| BY 29 (à 63 % de matière active) | 6 % |
| Eau | qsp 100 % |

Mode opératoire: on chauffe séparément à 70°C les phases huileuse et aqueuse, puis on émulsionne en dispersant sous agitation vigoureuse la phase aqueuse dans la phase huileuse.

On obtient ainsi une émulsion douce et riche à l'application qui apporte une sensation de bien-être immédiat.

### Exemple 3 : Emulsion hydratante

| *Phase huileuse :* | |
|---|---|
| Gel d'organopolysiloxane (exemple 3 du brevet US-5,412,004) | 10 % |
| (soit environ 3,3 % de matière active) | |
| Huile de vaseline | 10 % |
| Cyclopentaméthicone | 10 % |
| Isohexadécane | 5 % |
| Beurre de shorea | 1 % |

| *Phase aqueuse :* | |
|---|---|
| Glycérine | 7 % |
| Sulfate de magnésium | 0,5 % |
| Conservateurs | 0,4 % |
| BY 29 (à 63 % de matière active) | 3 % |
| Eau | qsp 100 % |

Mode opératoire: on chauffe séparément à 70°C les phases huileuse et aqueuse, puis on émulsionne en dispersant sous agitation vigoureuse la phase aqueuse dans la phase huileuse.

On obtient ainsi une émulsion souple, agréable et efficace.

## Revendications

1. Composition sous forme d'émulsion eau-dans-huile, comprenant une phase aqueuse dispersée dans une phase huileuse, **caractérisée en ce qu'**elle contient au moins un tensioactif de HLB inférieur ou égal à 7, **en ce que** la phase huileuse représente au moins 5 % en poids par rapport au poids total de la composition, et **en ce que** la phase aqueuse contient une suspension aqueuse de particules d'organopolysiloxane élastomère au moins partiellement réticulé.

2. Composition selon la revendication 1, **caractérisée en ce que** le tensioactif est choisi parmi les organopolysiloxanes oxyalkylénés, les alkylpolyglycosides, les esters d'acide gras et de polyol, et leurs mélanges.

3. Composition selon la revendication 1 ou 2, **caractérisée en ce que** le tensioactif est choisi parmi les dimethicone copolyols, les alkyldiméthicone copolyols, les organopolysiloxanes solides élastomères réticulés comportant au moins un groupement oxyalkyléné, l'isostéaryl-glucoside, l'oléyl-glucoside et leurs mélanges.

4. Composition selon l'une quelconque des revendications précédentes, **caractérisée en ce que** la quantité de tensioactif(s) va de 0,5 à 20 % en poids de matière active, par rapport au poids total de la composition.

5. Composition comprenant une phase aqueuse et une phase huileuse, l'une des phases étant dispersée dans l'autre, **caractérisée en ce qu'**elle contient au moins un organopolysiloxane solide élastomère réticulé comportant au moins un groupement oxyalkyléné, et **en ce que** la phase aqueuse contient une suspension aqueuse de particules d'organopolysiloxane élastomère au moins partiellement réticulé.

6. Composition selon la revendication précédente, **caractérisée en ce que** la quantité d'organopolysiloxane solide élastomère réticulé comportant au moins un groupement oxyalkyléné va de 0,5 à 20 % en poids de matière active, par rapport au poids total de la composition.

7. Composition selon l'une quelconque des revendications précédentes, **caractérisée en ce que** l'organopolysiloxane élastomère de la suspension aqueuse est obtenu par réaction d'addition et de réticulation, en présence d'un catalyseur, d'au moins :
- un organopolysiloxane (i) ayant deux groupements vinyliques en position α-ω de la chaîne siliconée par molécule ; et
- un organosiloxane (ii) ayant au moins un atome d'hydrogène lié à un atome de silicium par molécule.

8. Composition selon la revendication précédente, **caractérisée en ce que** l'organopolysiloxane (i) est choisi parmi les polydiméthylsiloxanes.

9. Composition selon la revendication 7 ou 8, **caractérisée en ce que** l'organopolysiloxane (i) est un α-ω-diméthylvinyl polydiméthylsiloxane.

10. Composition selon l'une quelconque des revendications 7 à 9, **caractérisée en ce que** la suspension aqueuse de particules d'organopolysiloxane est obtenue selon les étapes suivantes :
- (a) mélange de l'organopolysiloxane (i) et de l'organosiloxane (ii) ;
- (b) ajout de la phase aqueuse contenant un émulsifiant au mélange de l'étape (a) ;
- (c) émulsification de la phase aqueuse et dudit mélange ;
- (d) ajout d'eau chaude à l'émulsion de la phase (c) ; et
- (e) polymérisation de l'organopolysiloxane (i) et de l'organosiloxane (ii) en émulsion en présence d'un catalyseur de platine.

11. Composition selon la revendication précédente, **caractérisée en ce que** l'étape (c) est obtenue en présence d'un émulsifiant non-ionique.

12. Composition selon l'une quelconque des revendications précédentes, **caractérisée en ce que**, dans la suspension aqueuse, les particules d'organosiloxane élastomère ont une taille allant de 0,1 à 500 µm.

13. Composition selon l'une quelconque des revendications précédentes, **caractérisée en ce que** les particules d'organosiloxane de la suspension aqueuse sont présentes en une quantité en matière'active allant de 0,1 à 30 % en poids par rapport au poids total de la composition.

14. Composition selon l'une quelconque des revendications précédentes, **caractérisée en ce qu'**elle comporte de 10 à 80 % en poids de phase huileuse par rapport au poids total de la composition.

15. Composition selon l'une quelconque des revendications précédentes, **caractérisée en ce que** la phase huileuse contient au moins une huile minérale.

16. Composition selon l'une quelconque des revendications précédentes, **caractérisée en ce qu'**elle contient un actif choisi parmi les filtres solaires ; les vitamines et notamment les vitamines A, C, E, B3, F et D et leurs dérivés ; les acides gras insaturés ; l'alpha-bisabolol ; les beurres d'origine végétale ; l'urée ; la rutine ; les enzymes ; les extraits naturels tels que le thé vert, l'extrait de mélisse, l'extrait de thym, les oligomères procyannidoliques ; l'acide kojique ; l'acide caféique ; l'acide rétinoique et ses dérivés ; l'acide benzène 1,4-di-(3-méthylidène 10-camphosulfonique) ; les caroténoïdes, et leurs mélanges.

17. Composition selon l'une quelconque des revendications précédentes, **caractérisée en ce qu'**elle constitue une composition cosmétique.

18. Utilisation cosmétique d'une composition cosmétique selon l'une quelconque des revendications 1 à 17, pour le traitement, la protection, le soin, le démaquillage et/ou le nettoyage de la peau, des lèvres et/ou des cheveux.

19. Procédé de traitement cosmétique de la peau, des cheveux, et/ou des lèvres, **caractérisé par le fait qu'**on applique sur la peau, les cheveux et/ou les lèvres, une composition cosmétique selon l'une quelconque des revendications 1 à 17.

20. Utilisation de la composition selon l'une quelconque des revendications 1 à 17, pour la fabrication d'une composition destinée au soin des peaux sèches et/ou des lèvres sèches et/ou des peaux sensibles.

21. Utilisation d'une suspension aqueuse de particules d'un d'organopolysiloxane élastomère au moins partiellement réticulé, dans la phase aqueuse d'une composition contenant au moins 5 % en poids de phase huileuse, par rapport au poids total de la composition, afin d'éliminer le toucher collant et/ou gras dû à cette phase huileuse.

## Patentansprüche

1. Zusammensetzung in Form einer Wasser-in-Öl-Emulsion, die eine in einer Ölphase dispergierte wässerige Phase aufweist, **dadurch gekennzeichnet, dass** sie mindestens einen grenzflächenaktiven Stoff mit einem HLB-Wert von kleiner oder gleich 7 enthält, dadurch, dass die Ölphase bezogen auf das Gesamtgewicht der Zusammensetzung mindestens 5 Gew.-% ausmacht, und dadurch, dass die wässerige Phase eine wässerige Suspension von Partikeln eines zumindest teilweise vernetzten elastomeren Polyorganosiloxans enthält.

2. Zusammensetzung nach Anspruch 1, **dadurch gekennzeichnet, dass** der grenzflächenaktive Stoff unter alkoxylierten Polyorganosiloxanen, Alkylpolyglykosiden, Estern von Fettsäuren und Polyolen und den Gemischen dieser Verbindungen ausgewählt ist.

3. Zusammensetzung nach Anspruch 1 oder 2, **dadurch gekennzeichnet, dass** der grenzflächenaktive Stoff unter Dimeticoncopolyolen, Alkyldimeticoncopolyolen, festen vernetzten elastomeren Polyorganosiloxanen, die mindestens eine alkoxylierte Gruppe aufweisen, I-sostearylglucosid, Oleylglucosid und den Gemischen dieser Verbindungen ausgewählt ist.

4. Zusammensetzung nach einem der vorhergehenden Ansprüche, **dadurch gekennzeichnet, dass** der als Wirkstoff ausgedrückte Mengenanteil des grenzflächenaktiven Stoffs (der grenzflächenaktiven Stoffe) im Bereich von 0,5 bis 20 Gew.-%, bezogen auf das Gesamtgewicht der Zusammensetzung, liegt.

5. Zusammensetzung, die eine wässerige Phase und eine Ölphase enthält, wobei eine der Phasen in der anderen Phase dispergiert ist, **dadurch gekennzeichnet, dass** sie mindestens ein festes vernetztes elastomeres Polyorganosiloxan enthält, das mindestens eine alkoxylierte Gruppe aufweist, und dadurch, dass die wässerige Phase eine wässerige Suspension von Partikeln eines zumindest teilweise vernetzten elastomeren Polyorganosiloxans enthält.

6. Zusammensetzung nach dem vorhergehenden Anspruch, **dadurch gekennzeichnet, dass** der als Wirkstoff ausgedrückte Mengenanteil des festen vernetzten elastomeren Polyorganosiloxans, das mindestens eine alkoxylierte Gruppe aufweist, im Bereich von 0,5 bis 20 Gew.-%, bezogen auf das Gesamtgewicht der Zusammensetzung, liegt.

7. Zusammensetzung nach einem der vorhergehenden Ansprüche, **dadurch gekennzeichnet, dass** das elastomere Polyorganosiloxan der wässerigen Suspension durch Addition und Vernetzung mindestens
- eines Polyorganosiloxans (i), das pro Molekül zwei Vinylgruppen in α-ω-Stellung der Siliconkette aufweist, und
- eines Organosiloxans (ii), das pro Molekül mindestens ein Wasserstoffatom aufweist, das an ein Siliciumatom gebunden ist, hergestellt wird, wobei die Umsetzung in Gegenwart eines Katalysators erfolgt.

8. Zusammensetzung nach dem vorhergehenden Anspruch, **dadurch gekennzeichnet, dass** das Polyorganosiloxan (i) unter den Polydimethylsiloxanen ausgewählt ist.

9. Zusammensetzung nach Anspruch 7 oder 8, **dadurch gekennzeichnet, dass** das Polyorganosiloxan (i) ein α-ω-Dimethylvinylpolydimethylsiloxan ist.

10. Zusammensetzung nach einem der Ansprüche 7 bis 9, **dadurch gekennzeichnet, dass** die wässerige Suspension von Polyorganosiloxan-Partikeln folgendermaßen hergestellt wird:
- (a) Mischen des Polyorganosiloxans (i) mit dem Organosiloxan (ii),
- (b) Zugabe der wässerigen Phase, die einen Emulgator enthält, zu dem Gemisch von Schritt (a),
- (c) Emulgieren der wässerigen Phase und des Gemischs,
- (d) Zugabe von warmem Wasser zu der Emulsion von Schritt (c) und
- (e) Polymerisation des in Emulsion befindlichen Polyorganosiloxans (i) und Organosiloxans (ii) in Gegenwart eines Platinkatalysators.

11. Zusammensetzung nach dem vorhergehenden Anspruch, **dadurch gekennzeichnet, dass** der Schritt (c) in Gegenwart eines nichtionischen Emulgators durchgeführt wird.

12. Zusammensetzung nach einem der vorhergehenden Ansprüche, **dadurch gekennzeichnet, dass** die elastomeren Organosiloxan-Partikel in der wässerigen Suspension eine Größe von 0,1 bis 500 µm aufweisen.

13. Zusammensetzung nach einem der vorhergehenden Ansprüche, **dadurch gekennzeichnet, dass** die Organosiloxan-Partikel der wässerigen Suspension in einem als Wirkstoff ausgedrückten Mengenanteil von 0,1 bis 30 Gew.-%, bezogen auf das Gesamtgewicht der Zusammensetzung, vorliegen.

14. Zusammensetzung nach einem der vorhergehenden Ansprüche, **dadurch gekennzeichnet, dass** sie 10 bis 80 Gew.-% Ölphase, bezogen auf das Gesamtgewicht der Zusammensetzung, enthält.

15. Zusammensetzung nach einem der vorhergehenden Ansprüche, **dadurch gekennzeichnet, dass** die Ölphase mindestens ein Mineralöl enthält.

16. Zusammensetzung nach einem der vorhergehenden Ansprüche, **dadurch gekennzeichnet, dass** sie einen Wirkstoff enthält, der unter Sonnenschutzfiltern, Vitaminen und insbesonders Vitamin A, C, E, B3, F und D und ihren Derivaten, ungesättigten Fettsäuren, α-Bisabolol, Butterarten pflanzlichen Ursprungs, Harnstoff, Rutin, Enzymen, natürlichen Extrakten, wie grünem Tee, Melissenextrakt und Thymianextrakt, Procyanidin-Oligomeren, Kojisäure, Kaffeesäure, Retinsäure und ihren Derivaten, Benzol-1,4-di-(3-methylidencampher-10-sulfonsäure), Carotinoiden und deren Gemischen ausgewählt ist.

17. Zusammensetzung nach einem der vorhergehenden Ansprüche, **dadurch gekennzeichnet, dass** sie eine kosmetische Zusammensetzung darstellt.

18. Kosmetische Verwendung einer kosmetischen Zusammensetzung nach einem der Ansprüche 1 bis 17 zur Behandlung, zum Schutz, zur Pflege, zum Abschminken und/oder zur Reinigung der Haut, der Lippen und/oder der Haare.

19. Verfahren zur kosmetischen Behandlung der Haut, der Haare und/oder der Lippen, **dadurch gekennzeichnet, dass** eine kosmetische Zusammensetzung nach einem der Ansprüche 1 bis 17 auf die Haut, die Haare und/oder die Lippen aufgebracht wird.

20. Verwendung der Zusammensetzung nach einem der Ansprüche 1 bis 17 zur Herstellung einer Zusammensetzung, die zur Pflege von trockener Haut und/oder trockenen Lippen und/oder empfindlicher Haut bestimmt ist.

21. Verwendung einer wässerigen Suspension von Partikeln eines zumindest teilweise vernetzten elastomeren Polyorganosiloxans in der wässerigen Phase einer Zusammensetzung, die bezogen auf das Gesamtgewicht der Zusammensetzung mindestens 5 Gew.-% einer Ölphase enthält, um das klebrige und/oder fettige Gefühl zu beseitigen, das von der Ölphase herrührt.

## Claims

1. Composition in the form of a water-in-oil emulsion, comprising an aqueous phase dispersed in an oily phase, **characterized in that** it contains at least one surfactant with an HLB value of less than or equal to 7, **in that** the oily phase represents at least 5% by weight relative to the total weight of the composition, and **in that** the aqueous phase contains an aqueous suspension of particles of an at least partially crosslinked elastomeric polyorganosiloxane.

2. Composition according to Claim 1, **characterized in that** the surfactant is chosen from oxyalkylenated polyorganosiloxanes, alkylpolyglycosides and fatty acid esters of polyols, and mixtures thereof.

3. Composition according to Claim 1 or 2, **characterized in that** the surfactant is chosen from dimethicone copolyols, alkyldimethicone copolyols, crosslinked solid elastomeric polyorganosiloxanes comprising at least one oxyalkylenated group, isostearyl-glucoside and oleyl-glucoside, and mixtures thereof.

4. Composition according to any one of the preceding claims, **characterized in that** the amount of surfactant(s) ranges from 0.5% to 20% by weight of active material, relative to the total weight of the composition.

5. Composition comprising an aqueous phase and an oily phase, one of the phases being dispersed in the other, **characterized in that** it contains at least one crosslinked solid elastomeric polyorganosiloxane comprising at least one oxyalkylenated group, and **in that** the aqueous phase contains an aqueous suspension of particles of an at least partially crosslinked elastomeric polyorganosiloxane.

6. Composition according to the preceding claim, **characterized in that** the amount of crosslinked solid elastomeric polyorganosiloxane comprising at least one oxyalkylenated group ranges from 0.5% to 20% by weight of active material, relative to the total weight of the composition.

7. Composition according to any one of the preceding claims, **characterized in that** the elastomeric polyorganosiloxane in the aqueous suspension is obtained by addition and crosslinking reaction, in the presence of a catalyst, of at least:
- one polyorganosiloxane (i) containing two vinyl groups in the α-ω position on the silicone chain per molecule; and
- one organosiloxane (ii) containing at least one hydrogen atom linked to one silicon atom per molecule.

8. Composition according to the preceding claim, **characterized in that** the polyorganosiloxane (i) is chosen from polydimethylsiloxanes.

9. Composition according to Claim 7 or 8, **characterized in that** the polyorganosiloxane (i) is an α,ω-dimethylvinyl polydimethylsiloxane.

10. Composition according to any one of Claims 7 to 9, **characterized in that** the aqueous suspension of polyorganosiloxane particles is obtained according to the following steps:
- (a) mixing the polyorganosiloxane (i) and the organosiloxane (ii);
- (b) adding the aqueous phase containing an emulsifier to the mixture from step (a);
- (c) emulsifying the aqueous phase and the said mixture;
- (d) adding hot water to the emulsion from phase (c); and
- (e) emulsion-polymerizing the polyorganosiloxane (i) and the organosiloxane (ii) in the presence of a platinum catalyst.

11. Composition according to the preceding claim, **characterized in that** step (c) is obtained in the presence of a nonionic emulsifier.

12. Composition according to any one of the preceding claims, **characterized in that**, in the aqueous suspension, the elastomeric organosiloxane particles have a size ranging from 0.1 µm to 500 µm.

13. Composition according to any one of the preceding claims, **characterized in that** the organosiloxane particles of the aqueous suspension are present in an active material content ranging from 0.1% to 30% by weight, relative to the total weight of the composition.

14. Composition according to any one of the preceding claims, **characterized in that** it comprises from 10% to 80% by weight of oily phase, relative to the total weight of the composition.

15. Composition according to any one of the preceding claims, **characterized in that** the oily phase contains at least one mineral oil.

16. Composition according to any one of the preceding claims, **characterized in that** it contains an active agent chosen from sunscreens; vitamins and especially vitamins A, C, E, B3, F and D and derivatives thereof; unsaturated fatty acids; α-bisabolol; butters of plant origin; urea; rutin; enzymes; natural extracts such as green tea, extract of balm, extract of thyme and procyannidol oligomers; kojic acid; caffeic acid; retinoic acid and its derivatives; benzene-1,4-bis(3-methylidene-10-camphorsulphonic acid); carotenoids, and mixtures thereof.

17. Composition according to any one of the preceding claims, **characterized in that** it constitutes a cosmetic composition.

18. Cosmetic use of a cosmetic composition according to any one of Claims 1 to 17, to treat, protect, care for, remove make-up from and/or cleanse the skin, the lips and/or the hair.

19. Cosmetic treatment process for the skin, the hair and/or the lips, **characterized in that** a cosmetic composition according to any one of Claims 1 to 17 is applied to the skin, the hair and/or the lips.

20. Use of the composition according to any one of Claims 1 to 17, for the manufacture of a composition intended for caring for dry skin and/or dry lips and/or sensitive skin.

21. Use of an aqueous suspension of particles of an at least partially crosslinked elastomeric polyorganosiloxane, in the aqueous phase of a composition containing at least 5% by weight of oily phase, relative to the total weight of the composition, in order to eliminate the sticky and/or greasy feel due to this oily phase.
